# EUROPEAN PATENT APPLICATION

(11) **EP 1 579 880 A1**
(43) Date of publication of application: **28.09.2005**
(21) Application number: 05003627.6
(22) Date of filing: 21.02.2005
(51) Int. Cl.: A61M 3/02

(54) **Medical apparatus for intestinal and vaginal treatments**

(30) Priority: 24.03.2004 IT MI20040571
(71) Applicant: BIEFFE S.R.L., 20147 Milan (IT)
(72) Inventor: Colombi, Allesandro, 20147 Milan (IT)
(74) Representative: Coloberti, Luigi

(57) **Abstract**

The self-irrigation medical apparatus for intestinal and/or vaginal irrigations, comprises a shaped frame (13) positionable between a toilet seat (12) and a toilet bowl (10), to support a disposable irrigation tube (14); the support frame (13) has a central portion (18) conformed to face the inside of the bowl (10), and two side arms (19, 20) which may be locked between opposite surfaces of the bowl (10) and the seat (12). A duct (21, 21') for the passage of a water flow, extends along the support frame (13), and into an upwardly facing pipe fitting (16), for the connection of the irrigation tube (14). The apparatus comprises a pressure control unit (22), and a manually actuable push bottom tap (36) on a side of the support frame (13).

## Description

### BACKGROUND OF THE INVENTION

This invention concerns a medical apparatus for self-irrigation treatments, for example for intestinal irrigations of the colon and vaginal irrigations, suitable for use both as a portable apparatus, and for permanent installation in the bathroom of a home or a hotel, for totally independent use, without the need for any assistance or help of an operator.

In particular, the invention is directed to a medical apparatus of the aforementioned kind, which can be used directly on toilet bowls, easily fitted and removed by a patient, and proves to be extremely easy and hygienic to use.

Although the medical apparatus is described hereunder with reference to hydrotherapeutic treatment of the colon, it is understood that the same apparatus can also be used for other types of irrigation, for example in the treatment of diseases or for vaginal douches.

### STATE OF THE ART

As described in a previous application EP-A-1 240 911, hydrotherapeutic treatment of the colon is a widely known technique, that has been used for many years for removing the faecal residues that normally line the walls of the colon.

In this connection, in order to be removed, the faecal lining of the colon must be thoroughly soaked and saturated with water, in such a way as to permit its gradual evacuation.

The controlled injection of a lavage fluid is normally carried out by means of special apparatuses operated by an expert operator, maintaining the patient in a recumbent position or lying on a bed; this involves the patient having to depend upon suitably equipped medical structures for such treatment.

In order to render the patient totally independent in carrying out and in handling the various operations of intestinal irrigation and lavage, US-A-2.007.069 and US-A-1.983.293 suggest the use of a special apparatus forming an integral part of the seat of a toilet bowl, in which an irrigation tube or specula extends from one edge of the seat towards the inside of the bowl and then upwards.

Although similar apparatuses have proved to be extremely useful, in that they have rendered the patient wholly independent in carrying out intestinal irrigation and/or lavage treatments, in practice they have a number of drawbacks.

In particular, the seat must be suitably conformed and provided with the necessary ducts for passage of the water, and means for supporting the irrigation tube; consequently, they call for a fixed installation which does not allow the apparatus to be transported or fitted onto another toilet bowl located elsewhere.

Even though the solution may seem to be apparently simple, from the hygienic standpoint the proposed solution is somewhat questionable, due to the difficulty in carrying out the necessary cleaning operations.

The total lack of any safety device or devices for controlling the incoming flow of water, especially whenever the apparatus is connected to a pressurised water supply, makes these apparatuses somewhat unpractical and wholly unsafe to use.

In order to obviate a large part of these drawbacks, EP-A-1.240.911 again suggests the use of a special apparatus as an integral part of the seat of a toilet bowl, again proving to be of fixed installation and not transportable.

Although an apparatus of this kind is provided with the necessary safety devices, and with accessories capable of enabling it to be connected to a water supply tap, in practice, the apparatus still cannot be used in a universal manner or under different conditions of use.

The safety and control unit for controlling the pressure and the water flow is positioned far from the toilet bowl, in an uncomfortable position for a patient, which does not allow any easy and urgent intervention of the same patient during an emergency.

In all the cases, the previously known apparatuses call for the need to make use of a specially dedicated and expensive seat, which is not always pleasant to see in the bathroom of a modern home, and do not contemplate the possibility of transporting the apparatus itself.

Lastly, US-A-5,946,741 describes an accessory that can be fitted onto a toilet bowl, for carrying out intestinal irrigations, which can be installed between the seat and the toilet bowl, at the time of use, and then removed and stored when not in use.

This apparatus consists in a base plate that can be fitted between the opposite rear edges of the seat and the toilet bowl, in a totally back position beneath the seat, to prevent the base plate from becoming soiled during use.

A flexible pipe for feeding the water is connected to a first pipe fitting in correspondence with the front edge of the base plate, between the seat and the bowl, close to the pipe fitting for a special irrigation tube.

Although in theory the position of the base plate and the irrigation tube can be varied with respect to the toilet bowl and the seat, in actual fact an adjustment of the position could be made within extremely restricted limits, also in relation to the numerous different shapes and types of bowls and seats currently in use.

In a way wholly corresponding to the previously known apparatuses, the irrigation tube is still fitted onto a tubular arm which extends overhanging towards the bottom of the bowl and then upwards after a wide curve; therefore sometimes it can be difficult to self-introduce the irrigation tube correctly into the anus, without causing an injury or possible lesions to the patient, due to the incorrect positioning of the apparatus and the possible bending of the tubular arm supporting the irrigation tube whenever the patient is hesitant or does not pay sufficient attention at the time of use.

In addition to the problem of greater hygiene and correct positioning of the irrigation tube in relation to the different requirements and physical characteristics of the patient, and besides the problem of safety, there are other requirements which have been wholly unsatisfied or which have not been sufficiently or completely solved by the previously known apparatuses.

One of the requirements most widely expected, consists in rendering the apparatus completely transportable and usable in any bathroom of a home or a hotel, in which the patient must reside for a given period of time, without having to give up using the apparatus.

Another problem related to the former, which has never been dealt with and solved with the previous apparatuses, concerns its universal use, and the possibility of connecting the apparatus to any source of water existing in a bathroom.

In fact, by an investigation it has been observed that the connection of the water supply line to the normal water taps or mixers is at times difficult if not impossible, due to the enormous variety of models of taps and mixers currently existing, unless special coupling devices are used.

Conversely, in almost all the bathrooms existing nowadays, there is a possibility of using the normal shower, as a source of water.

### OBJECTS OF THE INVENTION

The general object of this invention is to provide a medical apparatus for intestinal self-irrigation treatments or for other similar applications, improved in use, whereby it is possible to obviate the drawbacks and the limitations of the previously known apparatuses.

A further object of this invention is to provide a medical apparatus for self-irrigation treatments, suitable for use with any type of toilet bowl, whereby it is possible to adjust the position of the irrigation tube as desired, regardless of the shape and dimensions of the bowl and the type of seat; moreover, the possibility of connecting the apparatus to the flexible pipe of any shower, makes the same apparatus practically of general-purpose type.

A further object of this invention is to provide a medical apparatus, of the aforementioned kind, which is capable of being used by patients of any build, even particularly obese patients, thanks to its extremely practical and easy use.

A still further object of this invention is to provide a medical apparatus in which the patients themselves can easily change the position of the irrigation tube, and easily control and adjust the water flow during injection, while maintaining a comfortable and correct posture, which facilitates the irrigation treatment.

A further object of this invention is to provide a medical apparatus as previously defined which, in addition to offering a high safety degree, is also hygienically treatable, since the same water supply duct can be used to wash the apparatus, maintaining it on the toilet bowl after use.

A still further object of the invention is to provide a medical apparatus for irrigation treatments, which can be packed in the form of a "kit"; in this way it is possible to easily assemble and disassemble the various parts making up the apparatus, and to transport them housed into a special case.

### BRIEF DESCRIPTION OF THE INVENTION

According to the invention a medical apparatus for self-irrigation treatments, has been provided to be fitted between a toilet seat and a toilet bowl, the apparatus comprising a disposable irrigation tube to be connected to a pressurised water source by means of a water supply line, characterised by comprising a tubular support frame for the irrigation tube, said support frame having a shaped central portion to transversally extends inside the bowl, and two side arms to be locked between the seat and the toilet bowl;
a flow duct for the water extending from a side arm into the central portion of the support frame, to an upwardly facing pipe fitting for the irrigation tube; a first automatic relief valve connected to the flow duct, said automatic relief valve being positioned on the support frame to be oriented towards the inside of the bowl;
and in that the water supply line comprises a manually operable tap and a pressure control unit on one side of the support frame the apparatus comprising also an extendable water feeding pipe provided with a connecting element which can be screwed to a flexible pipe of a shower, or for connection to the water delivery mouth of a tap.

According to the invention, a kit for a portable medical apparatus, for intestinal and/or vaginal irrigation treatments, has been provided comprising the following components:
- at least one irrigation tube;
- a shaped frame for supporting the irrigation tube, comprising a central portion and side arms, one of said arms and the central portion of the support frame comprising a flow duct for the water and an upwardly facing pipe connector for fitting an irrigation tube on the central portion of the support frame;
- a pressure and water flow control unit;
- a spirally-wound extendable water supply pipe;
- a filter;
- pipe fittings for connection of the extendable water supply pipe to a flexible pipe of a shower or for connection to a water tap; and
- a case having recessed seats for housing in an disassembled condition the components of the apparatus.

### BRIEF DESCRIPTION OF THE DRAWINGS

These and further features of the medical apparatus according to this invention, will be more clearly evident from the following description, with reference to the accompanying drawings, in which:
Fig. 1 shows an overall view of the medical apparatus for intestinal treatments, according to the invention;
Fig. 2 shows an enlarged detail of Fig. 1;
Fig. 3 shows an enlarged detail of Fig. 2;
Fig. 4 shows a cross section of the supporting bracket for the pressure control unit, along the line 4-4 of Fig. 2;
Fig. 5 shows a cross-section of the support frame for the irrigation tube, along the line 5-5 of Fig. 2;
Fig. 6 shows an enlarged detail of Fig. 3, with the irrigation tube removed;
Fig. 7 shows an accessory for connecting the apparatus to a mixer unit for hot and cold water;
Fig. 8 shows an enlarged detail of Fig. 2.

### DETAILED DESCRIPTION OF THE INVENTION

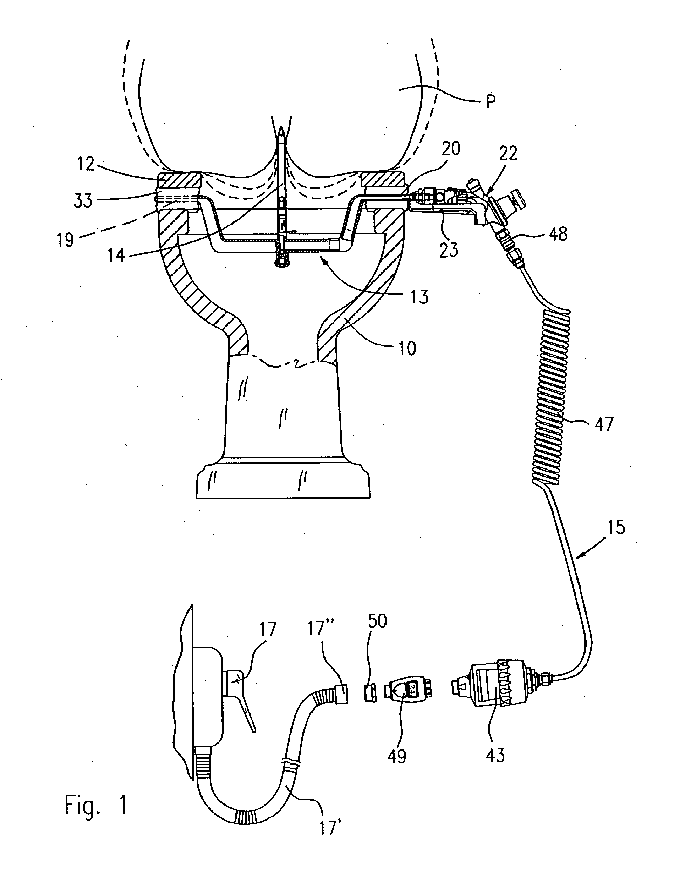

Fig. 1 shows the medical apparatus for self-irrigation treatments, according to the invention, fitted onto a normal toilet bowl 10, provided with a seat 12.

As shown in fig. 1 the medical apparatus according to the invention is positioned between the seat 12 and the bowl 10.

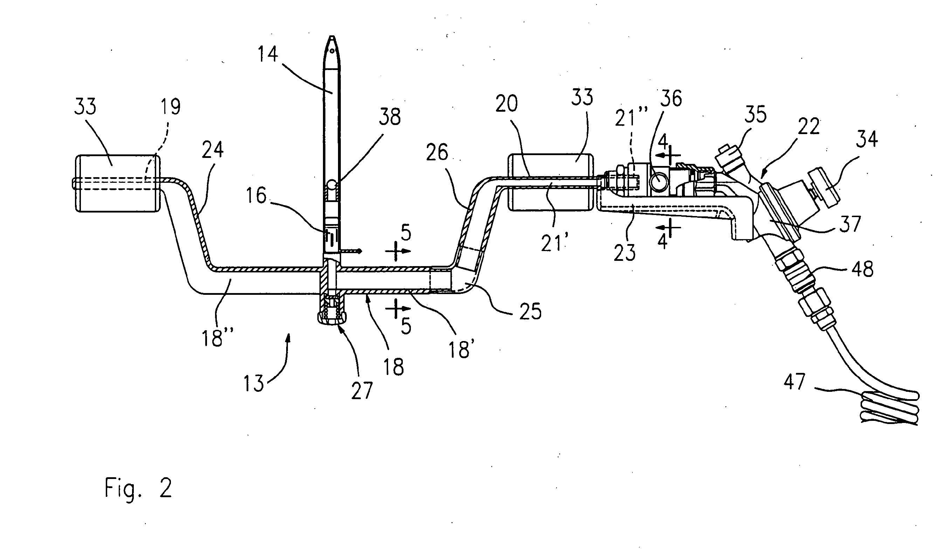

As more clearly illustrated in the enlarged detail of Fig. 2, the apparatus comprises a shaped support frame 13 for a disposable irrigation tube 14 which protrudes beyond the seat 12 to partially penetrate into the rectum of a patient P seated directly on the toilet bowl 10.

A water supplying line 15 for the irrigation tube 14, extends between an upwardly directed pipe fitting 16 for the irrigation tube 14, and a pressurised water source consisting for example of a mixer tap 17, preferentially provided with a flexible shower pipe 17'.

The aforesaid flexible shower pipe 17' is almost provided with a gas female pipe fitting 17", for the connection of a showerhead, not shown.

In order to ensure the possibility of using the medical apparatus in different places, for example at home, in a hotel or elsewhere, use has been made of an intermediate gas male ad female screw-type connecting element 50 for the operative connection between any type of flexible shower pipe 17' and the water supply line 15 of the apparatus itself, thereby achieving considerable advantages, which mainly consist in the possibility of using the apparatus in almost all type of bathrooms.

According to a preferential embodiment, the tubular support frame 13 comprises a shaped central portion 18 provided with the aforesaid pipe fitting 16 for removably supporting the irrigation tube 14; the central portion is the support frame 13, as shown in fig. 1, is conformed to extend transversally and towards the inside of the bowl 10; moreover, the support frame 13 comprises two side arms 19, 20 designed to be locked between opposite surfaces of the toilet bowl 10 and the seat 12, so as to maintain the irrigation tube 14 in a perfectly vertical position with respect to the sitting position of the patient P.

A similarly upstanding and perfectly centred disposition of the irrigation tube 14, with respect to the previously known solutions, in which the irrigation tube was supported or shaped to result overhanging and capable of laterally bending in the event of a not perfect posture of the patient, has the advantage of enormously facilitating the self-insertion of the irrigation tube 14 into the rectum, without causing damage or being excessively traumatic for the patient.

The support frame 13 can be made of moulded plastic material, for example polyester, or other suitable material or combination of different materials.

One 20 of the side arms and the central portion 18 on one side of the irrigation tube 14 a duct 21', 18' for the water, which operatively connects the pipe fitting 16 with a control unit 22 for controlling the pressure and flow of water, along the water supply line 15.

Said control unit 22 for the pressure and flow of water is connected to the duct 21' by means of a 21" for the feeding line, which extends beyond the side arm 20, and is supported on one side of the support frame 13 by a bracket 23 protruding from the side arm 20.

The support frame 13 for the irrigation tube 14 shown in the example of Fig. 2, is substantially trapezoidal in shape, and comprises a first and a second frame members fitted together; in particular, comprises a first frame member defining the central portion 18', provided with a bar shaped portion 18" which extends with a first diverging leg 24 ending with the side arm 19; on the other side the support frame 13 comprises a tubular section 18' defining part of the duct for passage of the water.

The first frame member 18' is removably connected to a second frame member having a second diverging leg 26, which extends into a side tubular arm 20 fastened to the supporting bracket 23.

The medical apparatus is also provided with pressure actuated safety valve means to relieve the pressure of the water, in order to avoid any injury in the event of incorrect regulation of the water pressure by the patient P; in particular, the support frame 13 comprises a first pressure relief valve 27, which is axially aligned to the pipe fitting 16 and is oriented in an opposite direction to the same pipe fitting 16 to discharge the pressurised water, downwards and inside the toilet bowl 10.

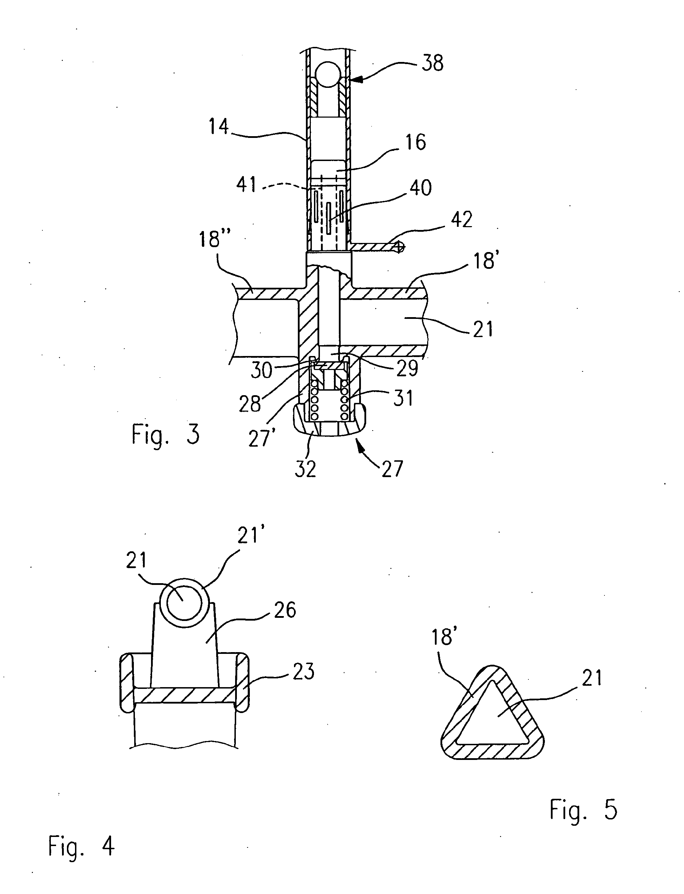

As shown in Fig. 3, said first relief valve 27 comprises a tubular protrusion 27' made in one piece with the central portion 18' of the support frame 13, within which slides a plug member 28 for closing a discharge hole 29 on one side of the water duct 21, for example in correspondence with the central portion 18' of the support frame 13.

The plug member 28 is urged against an annular seat 30 coaxially arranged to the discharge hole 29, by means of elastically yielding spring means 31, retained by a perforated cup 32 which can be screwed onto the tubular protrusion 27'.

Each side arm 19, 20 at the support frame 13 can be fitted with a sleeve 33 of elastically yielding material, for example rubber or other spongy material, to be disposed between the seat 12 and the upper edge of the bowl 10, thereby compensating for any differences of space between the opposite surfaces of the seat 12 and the bowl 10, and at the same time firmly locking the support frame 13 with the irrigation tube 14 upstanding in a correct vertical position.

The unit 22 for controlling the pressure and water flow comprises a manually operable pressure regulator 34, a pressure gauge for displaying the regulated pressure, not shown, a second automatic safety valve 35 and a pushbutton tap 36 which may be opened and closed by the same patient P to allow or stop the flow of water, along the water supply line 15, towards the irrigation pipe 14.

Preferentially, the pressure regulator 34, the pressure gauge and the second safety valve 35 are built in one body 37, while the pushbutton tap 36 is secured by screwing onto the body 37 of the control unit 22, in a very close position in respect to apparatus and the toilet bowl.

The pushbutton tap 36 is therefore disposed on one side of the pressure and flow control unit 22, facing towards the support frame 13 in order to be easily and conveniently operated by the patient P.

In order to ensure high standards of hygiene, the irrigation tube 14 preferably comprises, a one-way non-return valve 38, for example of ball-type, capable of preventing bacterial recycling due to a flow back of irrigation water towards the inside of the irrigation tube 14, following each operation for intestinal irrigation.

Moreover, the irrigation tube 14 is of the disposable type, in that its removal after use causes it to rupture.

In fact, as shown in figures 3, 6 and 7, the pipe fitting 16 for supporting the irrigation tube 14 has a cylindrical outer surface 39 provided with an annular sealing lip 16' and detent means for the irrigation tube 14; said detent means comprises a plurality of angularly spaced apart teeth 40 which extend longitudinally to the pipe fitting 16 for the irrigation tube 14; in order to prevent the irrigation tube 14 from slipping out once it has been threaded on the fitting 16, the detent teeth 40 increase in height rearwards towards the central portion 18' of the support frame 13, thereby defining shaped edges for retaining the tube 14.

The irrigation tube 14 can therefore be removed from the pipe fitting 16 exclusively by breaking the same irrigation tube 14 at its rear end; as shown in Fig. 3, this can be achieved by providing appropriately weakened lines 41 and a tear off tab 42 at the rear end of the irrigation tube 14 to facilitate the breaking of.

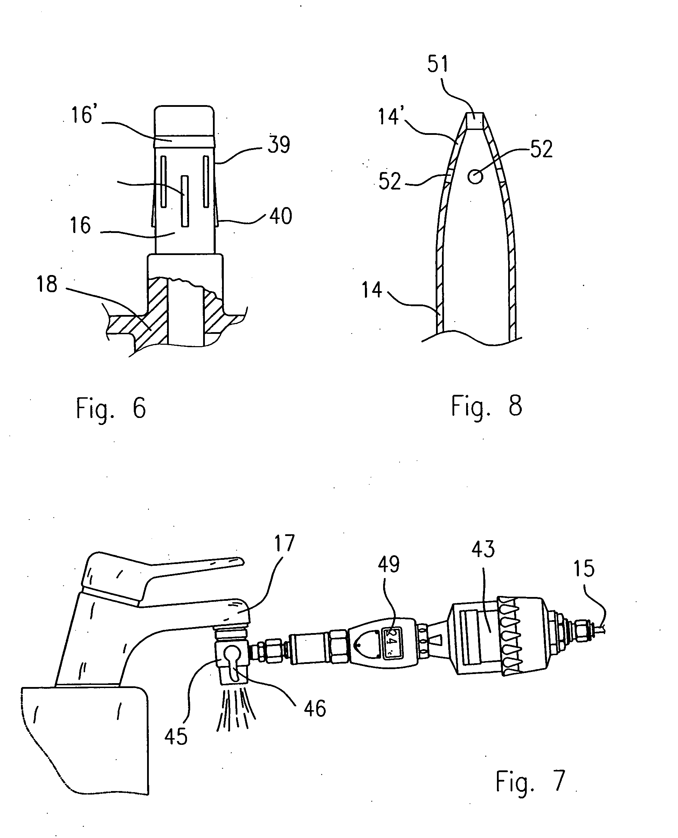

As shown in Fig. 8, the irrigation tube 14 at its upper end 14' is provided with some holes or slits for the outflow of the irrigation water.

Preferentially, in order to ensure a more efficient softening and removal of faecal residues from the inside of the intestine, the upper end 14' of the tube 14 may be provided, for example with an axial hole 51 and at least two side holes 52, which enable the water to provide a combined vertical and horizontal action, thereby optimising the therapeutic results.

In order to improve the quality of the water used for the irrigation, the apparatus is provided with a filter 43, for example a cartridge-type filter or of any other type, along the water supply line 15.

The connection of the apparatus to a water source can be achieved in any appropriate way; for example by means of a lever-type clamp, for clamping onto the delivery spout of a mixer tap 17 or, as shown in Fig. 7, by providing a manually operable valve 45, screwed onto or otherwise secured to the spout of the mixer tap 17 provided with a manually operable control lever 46.

Preferentially, the water supply line 15 comprises a spirally-wound extensible water-supply pipe 47, which offers considerable safety in the event of rupture or malfunctioning of the automatic discharge valves 27 and 35; in fact, by suitably choosing the length of the pipe 47, the pressure and flow rate of the water may be reduced thanks to the shape of the pipe 47 wound in the form of an helical "spring", causing a pressure drop depending on the length of the pipe 47.

The extensible pipe 47 is also provided with a quick-coupling connector 48 (fig. 1) to be connected to the pressure control unit 22, in such a way as to be easily disconnected and used for washing the apparatus, maintaining the same inside of the toilet bowl after use.

Moreover, all the components of the apparatus are preferably connected along the water supply line 15 by means of quick couplings or snap-on couplings, in order to allow a quick and easy assembling and disassembling of the same apparatus.

The apparatus can also comprise along the water supply line 15, a thermometer 49 normally available on the market, for controlling the temperature of the water; in fact, by means of the mixer tap 17 it is possible to adjust the temperature of the water supplied to irrigation tube 14, and to control the adjusted temperature by means of the thermometer 49.

Moreover, a manually-operated pump or other suitable accessory, not shown, can be provided along the water supply line 15 for injecting an aqueous solution containing, for example, a therapeutic composition or curative substance.

Fig. 4 shows a cross-sectional view of the supporting bracket 23 for the pressure control unit 22, in which it can be seen that the cross-section is preferentially H-shaped and capable of efficiently supporting and retaining the pressure control unit 22, offering good characteristics in terms of structural resistance.

Fig. 5 shows a cross-sectional view of the duct 21 for passage of the water provided by the support frame 13; as shown the duct 21 is preferentially triangular in shape.

The use of a self-irrigation apparatus according to the invention, in particular the use of a support frame which extends inside the bowl 10, as an alternative to the known apparatuses, offers several advantages, such as:
a) the possibility of using the apparatus with any type and shape of toilet bowl; in fact, by providing side arms 19, 20 of suitable length, it is possible to use the apparatus with bowls of different dimensions and widths;
b) the possibility of disposing the support frame 13 and consequently the irrigation tube 14, longitudinally in any position of the toilet bowl, in the most suitable condition for the patient.
   In fact, for adult patients or patients of heavy build, the frame 13 with the irrigation tube 14 can be located in a central position of the bowl, or in a slightly backward position; for young patients or patients of small build, the frame 13 and the irrigation tube 14 can be located in a more forward position.
c) Lastly, as shown in figure 1, the shape of the frame 13 extending towards the inside of the bowl 10, enables the apparatus to be used also by very obese patients, for whom in the past there was a certain amount of difficulty or impossibility of utilising the conventional type apparatuses for intestinal treatment.

The accompanying drawings show a preferential embodiment of a medical apparatus for intestinal and/or vaginal irrigation treatments according to the invention, in combination with a certain number of accessories which can be variously positioned along the line supplying the flow of water; it is obvious, however, that modification or variations to what has been shown are possible, both as regards the shape of the frame 13, and as regards the characteristics and/or disposition of the various accessories.

According to some tests carried out with patients of different types and with different body characteristics, it was observed that the apparatus is extremely practical and easy to use. In addition to being extremely simple, inexpensive and low cost, the proposed solution is also suitable for marketing in a kit by neatly arranging the various components in a suitable case, not shown, having recesses for housing the various parts of which the apparatus is composed.

It is understood therefore that what has been described and shown with reference to the accompanying drawings has been given purely by way of example in order to illustrate the general features of the medical apparatus for intestinal or vaginal treatments according to the invention, and one of its preferential embodiments, it remaining understood that other modifications or variations may be made, and that other components may be added, without thereby deviation from the scope of the accompanying claims.

## Claims

1. A medical apparatus for self-irrigation treatments, to be fitted between a toilet seat (12) and a toilet bowl (10), the apparatus comprising a disposable irrigation tube (14) to be connected to a pressurised water source (17) by means of a water supply line (15), **characterised by** comprising a tubular support frame (13) for the irrigation tube (14), said support frame (13) having a shaped central portion (18) to transversally extends inside the bowl (10), and two side arms (19, 20) to be locked between the seat (12) and the toilet bowl (10);
a flow duct (21) extending from a side arm (20) into the central portion of the support frame (13), to an upwardly facing pipe fitting for the irrigation tube (14);
a first automatic relief valve (27) connected to the flow duct (21), said automatic relief valve (27) being positioned on the support frame (13) to be oriented towards the inside of the bowl (10);
and in that the water supply line (15) comprises a manually operable tap (36) and a pressure control unit (22) on one side of the support frame (13).

2. The medical apparatus according to claim 1, **characterised by** comprising a sleeve (33) of elastically yielding material, fitted onto each side arm (19,20) of the support frame (13).

3. The medical apparatus according to claim 1, **characterised in that** the pressure control unit (22) comprises a pressure regulator (34), a pressure gauge, a second automatic safety valve (35) and a pushbutton tap (36) serially connected to the supply line (15).

4. The medical apparatus according to claim 3, **characterised in that** the pushbutton tap (36) is disposed on one side of the pressure control unit (22), facing towards the support frame (13).

5. The medical apparatus according to claim 1, **characterised in that** the irrigation tube (14) comprises a one-way non-return valve (38).

6. The medical apparatus according to claim 1, **characterised in that** the pipe fitting (16) for the irrigation tube (14) is provided with detent means (40) for tightly retaining the irrigation tube (14).

7. The medical apparatus according to claim 6, **characterised in that** said detent means for the irrigation tube (14) comprise a plurality of detent teeth (40) longitudinally extending on the pipe fitting (16) for the irrigation tube (14).

8. The medical apparatus according to claim 7, **characterised in that** the thickness of said detent teeth (40) increase in the direction of the support frame (13).

9. The medical apparatus according to claim 1, **characterised in that** the irrigation tube (14) has a tear off tab (42) and weakened breaking lines (41) at a rear end.

10. The medical apparatus according to claim 1, **characterised in that** said first relief valve (27) comprises a discharge hole (29) on one side of the flow duct (21) for the water, a plug member (28) for closing the discharge hole (29), and elastically yielding thrust means (31) to urge the plug member (28) against the discharge hole (29).

11. The medical apparatus according to claim 1, **characterised in that** the water supply line (15) comprises a filter (43).

12. The medical apparatus according to claim 1, **characterised in that** the water supply line (15) comprises a spirally-wound extensible tube (47), said extensible tube (47) being provided with a quick-coupling connector (48) for the pressure control unit (22).

13. The medical apparatus according to claim 1, **characterised by** comprising means for injecting a curative substance into the water supply line (15).

14. The medical apparatus according to claim 1, **characterised by** comprising a thermometer (49) for sensing the temperature of the water flowing through the water supply line (15).

15. The medical apparatus according to claim 1, **characterised by** comprising a water flow connecting element provided with a lever actuated clamp for connecting the water supply line (15) to a tap (17).

16. The medical apparatus according to claim 1, **characterised by** comprising a manually operable valve (45) for delivery of the water, respectively for connection to the water supply line (15).

17. A kit for containing and transporting a portable medical apparatus, for intestinal and/or vaginal irrigation treatments according to claim 1, comprising:
- at least one irrigation tube (14);
- a shaped frame (13) for supporting an irrigation tube (14), said support frame (13) comprising a central portion (18) and side arms (19, 20), one of said arms (20) and the central portion (18') of the support frame (13) comprising a flow duct (21) for the water and an upwardly facing pipe connector (16) for fitting an irrigation tube (14);
- a pressure and the water flow control unit (22);
- a spirally-wound extendable water supply pipe (47);
- a filter (43);
- pipe fittings (43, 49) for connection of the extendable supply pipe (47) to flexible pipe (17') of a shower, or for connection to a water tap (17); and
- a case having recessed seats for housing in an disassembled condition the components (13, 14, 22, 43, 47) of the apparatus.
